(19) Europäisches Patentamt
European Patent Office
Office européen des brevets

(11) **EP 3 498 257 A1**

(12) **EUROPEAN PATENT APPLICATION**

(43) Date of publication:
**19.06.2019 Bulletin 2019/25**

(21) Application number: **17206592.2**

(22) Date of filing: **12.12.2017**

(51) Int Cl.:
***A61K 8/46*** *(2006.01)*
***A61Q 5/00*** *(2006.01)*
***A61Q 13/00*** *(2006.01)*
***A61K 8/73*** *(2006.01)*
***A61Q 19/00*** *(2006.01)*
***A61K 8/87*** *(2006.01)*
***A61K 8/11*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD TN**

(71) Applicant: **The Procter & Gamble Company**
**Cincinnati, OH 45202 (US)**

(72) Inventors:
- **GUSKEY, Gerald**
  **Cincinnati, Ohio 45202 (US)**
- **WEI, Karl**
  **Cincinnati, Ohio 45202 (US)**

(74) Representative: **Sauvaître, Thibault Bruno**
**Procter & Gamble Service GmbH**
**IP Department**
**Sulzbacher Straße 40-50**
**65824 Schwalbach am Taunus (DE)**

# (54) COMPOSITIONS WITH POLYURETHANE MICROCAPSULES HAVING IMPROVED LONG-LASTING ODOR BENEFIT

(57) Described herein, a composition comprises an anionic surfactant system comprising from 1% to 30% of an anionic surfactant by total weight of the composition, wherein the anionic surfactant is sodium laureth(n) sulfate SLEnS, wherein n is the average moles of ethoxylation, wherein n ranges from 0 to 8, preferably from 1 to 3. The composition comprises from 0.01% to 2% of a cationic guar polymer by total weight of the composition, wherein the cationic guar polymer has a charge density from 0.6 meq/g to 2.5 meq/g. One or more coacervates are formed with the anionic surfactant system, preferably with the anionic surfactant, and the cationic guar polymer. The composition comprises from 0.001% to 10% of a plurality of polyurethane microcapsules by weight of the composition, wherein the polyurethane microcapsules comprise a shell material encapsulating a core material, said core material being disposed within said shell material, wherein said shell material comprises a polyurethane polymer and said core material comprises a benefit agent, wherein the polyurethane polymer of the shell material is the reaction product of the polymerization between one or more polyisocyanates and glycerine, and wherein the one or more polyisocyanates have at least two functional isocyanate groups. The one or more coacervates comprise one or more clusters. The one or more clusters includes a portion of the plurality of polyurethane microcapsules.

Fig. 1

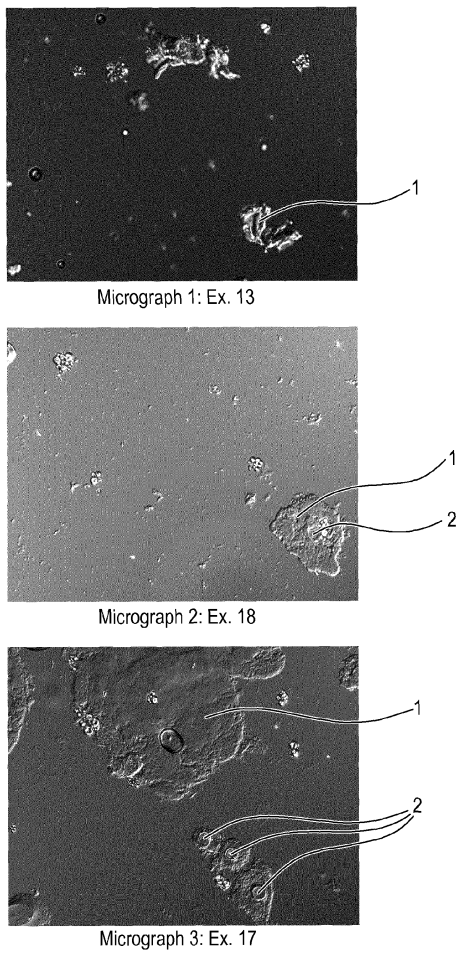

Micrograph 1: Ex. 13
Micrograph 2: Ex. 18
Micrograph 3: Ex. 17

EP 3 498 257 A1

Printed by Jouve, 75001 PARIS (FR)

## Description

FIELD OF THE INVENTION

The present application generally relates to compositions containing one or more coacervates formed with an anionic surfactant system and a cationic guar polymer. The composition comprises a plurality of polyurethane microcapsules, wherein the one or more coacervates comprise one or more clusters including a portion of the plurality of polyurethane microcapsules. The composition comprising the one or more coacervates including the portion of the plurality of polyurethane microcapsules have improved long-lasting odor benefit onto skin.

BACKGROUND OF THE INVENTION

The design of benefit delivery systems that can efficiently deposit from a composition which is in the form of a personal care cleaning composition onto hair or skin, or in the form of a liquid detergent on fabrics to provide a long-lasting perception of benefit agents such as volatile perfumes is a challenging task.
Benefit agents, such as perfumes, silicones, waxes, flavors, vitamins and fabric softening agents, are expensive and/or generally less effective when employed at high levels in consumer products, for example, personal care compositions, cleaning compositions, and fabric care compositions. As a result, there is a desire to maximize the effectiveness of such benefit agents.
Specific benefit agents such as perfumes and fragrances may delight the user by providing a freshness feeling and may serve as a signal to the user that the product may still be working or that the product is still present. Also, it is desired to provide a perfume benefit to fabrics when the fabrics are still wet from such treatment and after such fabrics have been dried.
Fragrances are typically highly volatile compounds that can readily evaporate during application. Because of the volatility of many fragrances, a consumer may be unable to notice the fragrance shortly after using the consumer product, potentially leading the user to believe the benefits are dissipating or have dissipated. Also, some other types of fragrances are prone to oxidation under ambient conditions leading to a rapid loss of their activity. To overcome these challenges, different alternatives can be used such as polymer profragrances, or polymer nanoparticles, or microcapsules as fragrance delivery systems. In particular, the encapsulation of fragrances in polymer core-shell microcapsules can prevent degradation of the benefit agent, avoid premature evaporation, and allow controlled release.
However, while much of the work has been done on polymer-based fragrance delivery systems to address issues related to the volatility, stability, and controlled release of fragrances, it is still an object to provide a composition with improved deposition of these systems on the surface of interest. Consumers today desire compositions that deposit and retain encapsulated benefit agents onto the surface of interest for an extended period of time.
Accordingly, there is a need to optimize the olfactive impact and benefits of fragrance delivery systems, their deposition and substantivity or retention after rinsing.
There is a need for a composition that provides an increased deposition of encapsulated benefit agents onto the surface of interest.
Furthermore, there is a need for a composition that provides an increased retention of encapsulated benefit agents onto the surface of interest for an extended period of time.

SUMMARY OF THE INVENTION

A composition is provided and comprises:

(a) an anionic surfactant system comprising from 1% to 30% of an anionic surfactant by total weight of the composition, wherein the anionic surfactant is sodium laureth(n) sulfate SLEnS, wherein n is the average moles of ethoxylation, wherein n ranges from 0 to 8, preferably from 1 to 3;
(b) from 0.01% to 2% of a cationic guar polymer by total weight of the composition, wherein the cationic guar polymer has a charge density from 0.6 meq/g to 2.5 meq/g;
(c) one or more coacervates formed with the anionic surfactant system, preferably with the anionic surfactant, and the cationic guar polymer;
(d) from 0.001% to 10% of a plurality of polyurethane microcapsules by weight of the composition, wherein the polyurethane microcapsules comprise a shell material encapsulating a core material, said core material being disposed within said shell material, wherein said shell material comprises a polyurethane polymer and said core material comprises a benefit agent, wherein the polyurethane polymer of the shell material is the reaction product of the polymerization between one or more polyisocyanates and glycerine, and wherein the one or more polyisocyanates have at least two functional isocyanate groups; and

wherein the one or more coacervates comprise one or more clusters, wherein the one or more clusters includes a portion of the plurality of polyurethane microcapsules.

Use of a composition comprising:

(a) an anionic surfactant system comprising from 1% to 30% of an anionic surfactant by total weight of the composition, wherein the anionic surfactant is sodium laureth(n) sulfate SLEnS, wherein n is the average moles of ethoxylation, wherein n ranges from 0 to 8, preferably from 1 to 3;
(b) from 0.01% to 2% of a cationic guar polymer by total weight of the composition, wherein the cationic guar polymer has a charge density from 0.6 meq/g to 2.5 meq/g;
(c) one or more coacervates formed with the anionic surfactant system, preferably with the anionic surfactant, and the cationic guar polymer; and
(d) from 0.001% to 10% of a plurality of polyurethane microcapsules by weight of the composition, wherein the polyurethane microcapsules comprise a shell material encapsulating a core material, said core material being disposed within said shell material, wherein said shell material comprises a polyurethane polymer and said core material comprises a benefit agent, wherein the polyurethane polymer of the shell material is the reaction product of the polymerization between one or more polyisocyanates and glycerine, and wherein the one or more polyisocyanates have at least two functional isocyanate groups,

wherein the one or more coacervates comprise one or more clusters, wherein the one or more clusters includes a portion of the plurality of polyurethane microcapsules;
for providing a long-lasting benefit on a surface of interest, preferably hair, skin or fabrics, more preferably hair or skin, for at least 1 hour, preferably at least 4 hours, and even at least 8 hours after initial use, preferably from 1 hour to 4 hours after initial use.
Use of the composition as set out hereinabove for improving the viscoelasticity of the one or more coacervates in a micellar surfactant system.

## BRIEF DESCRIPTION OF THE DRAWINGS

While the specification concludes with claims particularly pointing out and distinctly claiming the present invention, it is believed that the same will be better understood from the following description read in conjunction with the accompanying drawings in which:

FIG. 1 represents the micrographs corresponding to Examples 13, 18 and 17 generated by a light microscope (Zeiss AX10 Imager.A2 with 40X magnification from Carl Zeiss Co.; Goettengen, Germany); and
FIG. 2 represents the deposition index versus the charge density of the cationic guar polymers.

## DETAILED DESCRIPTION OF THE INVENTION

### Definitions and general

In this document, including in all embodiments of all aspects of the present invention, the following definitions apply unless specifically stated otherwise.

All percentages are by weight (w/w) of the respective composition, unless otherwise specified. All ratios or percentages are weight ratios or weight percentages unless specifically stated otherwise. "% wt." means percentage by weight. References to "parts" e.g. a mixture of 1 part X and 3 parts Y, is a ratio by weight. When more than one composition are used during a treatment, the total weight to be considered is the total weight of all the compositions applied on the hair simultaneously (i.e. the weight found "on head"), unless otherwise specified.

"QSP" or "q.s." means sufficient quantity for 100% or for 100g. "+/-" indicates the standard deviation. All ranges are inclusive and combinable. The number of significant digits conveys neither a limitation on the indicated amount nor on the accuracy of the measurement.

All measurements are understood to be made at 20°C and at ambient conditions, where "ambient conditions" means at 1 atmosphere (atm) of pressure and at 65% relative humidity, unless otherwise stated. "Relative humidity" refers to the ratio (stated as a percent) of the moisture content of air compared to the saturated moisture level at the same temperature and pressure. Relative humidity can be measured with a hygrometer, in particular with a probe hygrometer from VWR® International.

Herein "min" means "minute" or "minutes". Herein "mol" means mole. Herein "g" following a number means "gram" or "grams". "Ex." means "example". All amounts as they pertain to listed ingredients are based on the active level ("solids") and do not include carriers or by-products that may be included in commercially available materials.

Herein, "comprising" means that other steps and other ingredients can be included in addition. "Comprising" encompasses the terms "consisting of' and "consisting essentially of'. The compositions, methods, and uses of the present invention can comprise, consist of, and consist essentially of the elements and limitations of the invention described herein, as well as any of the additional or optional ingredients, components, steps, or limitations described herein. Embodiments and aspects described herein may comprise or be combinable with elements, features or components of other embodiments and/or aspects despite not being expressly exemplified in combination, unless an incompatibility is stated.

As used herein, the articles including "a" and "an" when used in a claim, are understood to mean one or more of what is claimed or described.

The terms "include," "includes," and "including," as used herein are meant to be non-limiting.

Where amount ranges are given, these are to be understood as being the total amount of said ingredient in the composition, or where more than one species fall within the scope of the ingredient definition, the total amount of all ingredients fitting that definition, in the composition.

For example, if the composition comprises from 1% to 5% fatty alcohol, then a composition comprising 2% stearyl alcohol and 1% cetyl alcohol and no other fatty alcohol, would fall within this scope.

The amount of each particular ingredient or mixtures thereof described hereinafter can account for up to 100% (or 100%) of the total amount of the ingredient(s) in the composition.

The term "substantially free of' as used herein means less than 1%, less than 0.8%, less than 0.5%, less than 0.3%, or less than an immaterial amount of by total weight of the composition.

The term "consumer product" as used herein means conditioner products intended to be used or consumed in the form in which it is sold. Such products include but are not limited to products for and/or uses relating to treating as a surface of interest hair, skin or fabrics. As used herein "consumer product" means baby care, beauty care, fabric & home care, family care, feminine care, health care, snack and/or beverage products or devices intended to be used or consumed in the form in which it is sold, and not intended for subsequent commercial manufacture or modification. Such products include but are not limited to fine fragrances (e.g. perfumes, colognes, eau de toilettes, after-shave lotions, pre-shave, face waters, tonics, and other fragrance-containing compositions for application directly to the skin), diapers, bibs, wipes; products for and/or methods relating to treating hair (human, dog, and/or cat), including, bleaching, coloring, dyeing, conditioning, shampooing, styling; deodorants and antiperspirants; personal cleansing; cosmetics; skin care including application of creams, lotions, and other topically applied products for consumer use; and shaving products, products for and/or methods relating to treating fabrics, hard surfaces and any other surfaces in the area of fabric and home care, including: air care, car care, dishwashing, fabric conditioning (including softening), laundry detergency, laundry and rinse additive and/or care, hard surface cleaning and/or treatment, and other cleaning for consumer or institutional use; products and/or methods relating to bath tissue, facial tissue, paper handkerchiefs, and/or paper towels; tampons, feminine napkins; products and/or methods relating to oral care including toothpastes, tooth gels, tooth rinses, denture adhesives, tooth whitening; over-the-counter health care including cough and cold remedies, pain relievers, RX pharmaceuticals, pet health and nutrition, and water purification; processed food products intended primarily for consumption between customary meals or as a meal accompaniment (non-limiting examples include potato chips, tortilla chips, popcorn, pretzels, corn chips, cereal bars, vegetable chips or crisps, snack mixes, party mixes, multigrain chips, snack crackers, cheese snacks, pork rinds, corn snacks, pellet snacks, extruded snacks and bagel chips); and coffee.

The term "cleaning composition" as used herein includes, unless otherwise indicated, granular or powder-form all-purpose or "heavy-duty" washing agents, especially cleaning detergents; liquid, gel or paste-form all-purpose washing agents, especially the so-called heavy-duty liquid types; liquid fine-fabric detergents; hand dishwashing agents or light duty dishwashing agents, especially those of the high-foaming type; machine dishwashing agents, including the various pouches, tablet, granular, liquid and rinse-aid types for household and institutional use; liquid cleaning and disinfecting agents, including antibacterial hand-wash types, cleaning bars, mouthwashes, denture cleaners, dentifrice, car or carpet shampoos, bathroom cleaners; hair shampoos and hair-rinses; shower gels and foam baths and metal cleaners; as well as cleaning auxiliaries such as bleach additives and "stain-stick" or pre-treat types, substrate-laden products such as dryer added sheets, dry and wetted wipes and pads, nonwoven substrates, and sponges; as well as sprays and mists.

The term "fabric care composition" as used herein includes, unless otherwise indicated, fabric softening compositions, fabric enhancing compositions, fabric freshening compositions and combinations thereof. The form of such compositions includes liquids, gels, beads, powders, flakes, and granules.

The terms "microcapsule" or "encapsulated benefit agents" as used herein refers to polyurethane microcapsules.

The term "copolymer" as used herein refers to a polymer derived from two or more polymerizable monomers. When used in generic terms, the term "copolymer" is also inclusive of more than two distinct monomers, for example, terpolymers.

The term "mixtures" as used herein is meant to include a simple combination of materials and any compounds that may result from their combination.

The term "molecular weight" or "M.Wt." as used herein refers to the weight average molecular weight unless

otherwise stated. The weight average molecular weight can be measured by gel permeation chromatography ("GPC").

The term "surface of interest" as used herein refers to hair when the composition is a cleaning composition in the form of a hair shampoo, or a hair conditioner; alternatively to skin when the cleaning composition is in the form of a personal care product such as a shower or bath cream, a body wash or foaming body wash, a deodorant or antiperspirant consumer product; alternatively to fabrics such as a liquid detergent or a fabric softener.

The term "coacervate" as used herein is a unique type of electrostatically-driven liquid-liquid phase separation, resulting from association of oppositely charged macro-ions in certain conditions. This combination of oppositely charged macro-ions when they contact each other form a mass of precipitated complex.

The term "cluster" as used herein is an aggregation of a portion of the plurality of polyurethane microcapsules of the composition. A coacervate comprises one or more clusters, wherein the one or more clusters includes a portion of the plurality of polyurethane microcapsules.

**Benefits of the one or more coacervates**

While microencapsulation can provide chemical stability and allows control of the release of the benefit agent such as volatile fragrance, tuning the interactions of these delivery systems with the substrate of interest is equally important.

It is an object of the present invention to enhance surface deposition of the benefit agents and the corresponding delivery systems from a composition onto the surface of interest.

It is an object of the present invention to enhance the deposition of the fragrances and fragrance delivery systems such as polyurethane microcapsules from a cleaning composition onto hair or skin.

It is an object of the present invention to enhance the deposition of the fragrances and fragrance delivery systems such as polyurethane microcapsules from a fabric care composition onto fabrics.

It is an object of the present invention is to provide a composition that provides an increased deposition of encapsulated benefit agents onto a surface of interest.

A further object of the invention is to enhance the formation of one or more coacervates in a micellar system.

A further object of the invention is to provide a composition that provides an improved long-lasting odor benefit of encapsulated benefit agents onto the hair or skin for an extended period of time.

The objects of the present invention are to provide compositions and uses of the compositions as described in the Summary or as described hereinbelow for fulfilling these technical effects or goals. These objects and other advantages as may be apparent to those skilled in the art can be achieved through the present invention, which is described in the above Summary of the Invention and Detailed Description of the invention and which is defined in the claims which follow.

This is a challenging task due to the specific functional and physiochemical characteristics of each type of composition. For instance, the primary function of a cleaning composition such as a hair shampoo or a body wash is to cleanse respectively the hair fibers or the skin by removing hydrophobic molecules such as noncovalently bound lipids and dust particles. The removal of these hydrophobic molecules is typically achieved using an anionic surfactant system comprising anionic surfactants, as they can effectively entrap these molecules by forming micelles. These micelles can be easily rinsed off with water owing to the electrostatic repulsions between the anionic headgroup of the surfactants and the hair or skin surface.

Since most fragrances are hydrophobic, they will predominantly localize inside the micelles rather than in the aqueous phase. Consequently, most fragrances are rinsed off together with these surfactants. Enhancing the affinity for and deposition of the fragrance delivery systems on the substrate of interest could reduce this loss and contribute to a longer lasting perception.

Cleaning compositions are often comprised of a cationic polymer and an anionic surfactant, which via nonspecific electrostatic interactions deposit on hair or skin as a coacervate during rinsing upon dilution below the critical micelle concentration. Hence, the formation of such coacervate within a micellar system had been either difficult to achieve or has provided any benefit agent onto hair or skin significantly.

It has been surprisingly found that the coacervated formation in a micellar system can be promoted by the type of the cationic polymer and the charge density of the cationic polymer.

Furthermore, it has been surprisingly found that in that case, the coacervated formation is accompanied with the development of clusters of polyurethane microcapsules within the one or more coacervates. It has been found that the increase of the number of clusters of polyurethane microcapsules within the one or more coacervates can help for improving the deposition of polyurethane microcapsules delivered to a situs via a composition such as a cleansing composition. In this regard, the formation of clusters of polyurethane microcapsules within the one or more coacervates of the micellar composition containing polyurethane microcapsules may improve the deposition of the polyurethane microcapsules, and thereby lead to provide the benefit agent, e.g. a more noticeable bloom from the polyurethane microcapsules.

It has been found that the formation of clusters of polyurethane microcapsules may be enhanced by combining an anionic surfactant system with a cationic guar polymer having a specific charge density from 0.6 meq/g to 2.5 meq/g. It results in an improved deposition of the polyurethane microcapsules onto hair or skin, or onto fabrics; and thus an increased retention of fragrance components originating from polyurethane microcapsules deposited onto hair or skin. Also, these properties result in an improved olfactive performance as illustrated by the experimental part hereinbelow.

It is believed that the composition comprising polyurethane microcapsules, along with a coacervate as defined more in details hereinbelow can help to deliver an improved long-lasting odor benefit than compositions containing only polyurethane microcapsules even over an extended period of time. Indeed, the composition can provide a long-lasting benefit on a surface of interest, preferably hair, skin or fabrics, more preferably hair or skin. The composition can provide a long-lasting fragrance benefit onto a surface of interest, e.g. skin for at least 1 hour after initial use, preferably up to 4 hours or longer after initial use, more preferably from 1 hour to 4 hours after initial use. The composition may also be used for improving the viscoelasticity of the one or more coacervates in a micellar surfactant system.

DETAILED DESCRIPTION OF THE INVENTION

## ANIONIC SURFACTANT SYSTEM

A composition comprises an anionic surfactant system. The anionic surfactant system comprises from 1% to 30% of an anionic surfactant, preferably from 3% to 25% of an anionic surfactant, more preferably from 10% to 25% of an anionic surfactant by total weight of the composition. The anionic surfactant comprises sodium laureth(n) sulfate, hereinafter SLEnS, wherein n is the average moles of ethoxylation. n ranges from 0 to 8, preferably from 1 to 3.

It is understood that a material such as SLEnS, for example, can comprise a significant amount of molecules which have no ethoxylate, 1 mole ethoxylate, 2 mole ethoxylate, 3 mole ethoxylate, and so on in a distribution which can be broad, narrow or truncated. For example, SLE1S can comprise a significant amount of molecules which have no ethoxylate, 1 mole ethoxylate, 3 mole ethoxylate, and so on in a distribution which can be broad, narrow or truncated and still comprise SLE1S where the average of the distribution is 1.

The anionic surfactant system may comprise from 1% to 10% of an additional anionic surfactant, preferably from 3% to 9% of an additional anionic surfactant, more preferably from 5% to 9% of an additional anionic surfactant by total weight of the composition. The additional anionic surfactant may be selected from the group consisting of ammonium laureth sulfate, ammonium lauryl sulfate, triethylamine lauryl sulfate, triethylamine laureth sulfate, triethanolamine lauryl sulfate, triethanolamine laureth sulfate, monoethanolamine lauryl sulfate, monoethanolamine laureth sulfate, diethanolamine lauryl sulfate, diethanolamine laureth sulfate, lauric monoglyceride sodium sulfate, potassium laureth sulfate, sodium lauryl sarcosinate, sodium lauroyl sarcosinate, lauryl sarcosine, cocoyl sarcosine, ammonium cocoyl sulfate, sodium cocoyl isethionate, ammonium lauroyl sulfate, sodium cocoyl sulfate, sodium lauryl sulfate, potassium cocoyl sulfate, potassium lauryl sulfate, monoethanolamine cocoyl sulfate, sodium tridecyl benzene sulfonate, sodium dodecyl benzene sulfonate, and mixtures thereof.

The composition may comprise from 0.1% to 20% of a cosurfactant, preferably from 0.5% to 15% of a cosurfactant, more preferably from 1% to 10% of a cosurfactant by total weight of the composition. The cosurfactant may be selected from the group consisting of amphoteric surfactants, zwitterionic surfactants, and mixtures thereof.

The composition may include at least one of an amphoteric surfactant and a zwitterionic surfactant. Suitable amphoteric or zwitterionic surfactants can include those described in U.S. Patent No. 5,104,646 and U.S. Patent No. 5,106,609.

Amphoteric surfactants can include those that can be broadly described as derivatives of aliphatic secondary and tertiary amines in which an aliphatic radical can be straight or branched chain and wherein an aliphatic substituent can contain from 8 to 18 carbon atoms such that one carbon atom can contain an anionic water solubilizing group, e.g., carboxy, sulfonate, sulfate, phosphate, or phosphonate. Examples of compounds falling within this definition can be sodium 3-dodecyl-aminopropionate, sodium 3-dodecylaminopropane sulfonate, sodium lauryl sarcosinate, N-alkyltaurines such as the one prepared by reacting dodecylamine with sodium isethionate according to the teaching of U.S. Patent No. 2,658,072, N-higher alkyl aspartic acids such as those produced according to the teaching of U.S. Patent No. 2,438,091, and products described in U.S. Patent No. 2,528,378. The cosurfactant included in the composition described herein may comprise an amphoteric surfactant that is selected from the group consisting of sodium lauroamphoacetate, sodium cocoamphoacetate, disodium lauroamphoacetate, disodium cocodiamphoacetate, and mixtures thereof.

Zwitterionic surfactants suitable for use in the cosurfactant of the composition described herein may include those that are broadly described as derivatives of aliphatic quaternary ammonium, phosphonium, and sulfonium compounds, in which the aliphatic radicals can be straight or branched chains, and wherein one of the aliphatic substituents can contain from 8 to 18 carbon atoms and one can contain an anionic group, e.g., carboxy, sulfonate, sulfate, phosphate, or phosphonate. The zwitterionic surfactant included in the composition described herein may include one or more

betaines, including cocoamidopropyl betaine.

Alternatively, the amphoteric or zwitterionic surfactant may be selected from cocamidopropyl betaine, lauramidopropyl betaine, coco betaine, lauryl betaine, cocoamphoacetate, cocoamphodiacetate, lauroamphoacetate, lauroamphodiacetate, lauramine oxide, sarcosinate, glutamate, lactate and mixtures thereof.

The anionic surfactant system may further comprise one or more zwitterionic surfactants and the zwitterionic surfactant may be a co-surfactant selected from the group consisting of: lauryl hydroxysultaine, cocamidopropyl hydroxysultaine, coco-betaine, coco-hydroxysultaine, coco-sultaine, lauryl betaine, lauryl sultaine, and mixtures thereof.

Examples of betaine zwitterionic surfactants may include coco dimethyl carboxymethyl betaine, cocoamidopropyl betaine (CAPB), cocobetaine, lauryl amidopropyl betaine (LAPB), oleyl betaine, lauryl dimethyl carboxymethyl betaine, lauryl dimethyl alphacarboxyethyl betaine, cetyl dimethyl carboxymethyl betaine, lauryl bis-(2-hydroxyethyl) carboxymethyl betaine, stearyl bis-(2-hydroxypropyl) carboxymethyl betaine, oleyl dimethyl gamma-carboxypropyl betaine, lauryl bis-(2-hydroxypropyl)alpha-carboxyethyl betaine, and mixtures thereof. Examples of sulfobetaines may include coco dimethyl sulfopropyl betaine, stearyl dimethyl sulfopropyl betaine, lauryl dimethyl sulfoethyl betaine, lauryl bis-(2-hydroxyethyl) sulfopropyl betaine and mixtures thereof.

The zwitterionic surfactant can comprise or consist of cocamidopropyl betaine (CAPB), lauramidopropyl betaine (LAPB), and combinations thereof.

The cosurfactant of the composition may comprise a zwitterionic surfactant, preferably a betaine, more preferably cocamidopropyl betaine.

The anionic surfactant system may further comprise one or more non-ionic surfactants, wherein the one or more non-ionic surfactants is selected from cocoamide monoethanolamine, lauramide monoethanolamine, cocoyl glucoside, lauroyl glucoside, decyl glucoside, and other alkyl glucosides.

The composition may preferably not comprise any oil and/or lipid ingredients. The composition may preferably not comprise any oil selected from the group consisting of olive oil, sunflower oil, soybean oil, peanut oil, rapeseed oil, almond oil, palm oil, coconut oil, palm kernel oil, and mixtures thereof. The composition may preferably not comprise any ingredient selected from the group consisting of castor oil, mineral oil, paraffin oil, petrolatum oil, lanolin and derivatives thereof, volatile and non-volatile organosiloxanes, waxes, montan wax, ceresine, microcrystalline wax, hydroxyoctacosanyl hydroxystearate, beeswax, synthetic beeswax and silicone wax.

The composition may not preferably comprise any structuring anionic surfactant selecting from the group consisting of sodium trideceth(n) sulfate STnS wherein n is between 0 and 3, sodium tridecyl sulfate, sodium $C_{12}$-$_{13}$ alkyl sulfate, sodium $C_{12}$-$_{15}$ alkyl sulfate, sodium $C_{11}$-$_{15}$ alkyl sulfate, sodium $C_{12}$-$_{18}$ alkyl sulfate, sodium $C_{10}$-$_{16}$ alkyl sulfate, sodium $C_{12}$-$_{13}$ pareth sulfate, sodium $C_{12}$-$_{13}$ pareth-*n* sulfate, sodium $C_{12}$-$_{14}$ pareth-*n* sulfate, and mixtures thereof.

## CATIONIC GUAR POLYMER

A composition comprises a cationic guar polymer. The cationic guar polymer can allow the formation of one or more coacervates. As can be appreciated, the cationic charge of the cationic guar polymer can interact with an anionic charge of the anionic surfactant of the anionic surfactant system, to form the one or more coacervates.

The composition comprises from 0.01% to 2%, preferably from 0.05% to 1.5%, more preferably from 0.1% to 1%, or even more preferably from 0.15% to 0.5% of a cationic guar polymer by weight of the composition.

The cationic guar polymer has a charge density from 0.6 meq/g to 2.5 meq/g, preferably from 0.75 meq/g to 2.0 meq/g, more preferably from 1.0 meq/g to 1.75 meq/g.

The charge densities may be measured at the pH of intended use of the composition. (e.g., at pH 3 to pH 9; preferably at pH 4 to pH 8). The weight average molecular weight of cationic guar polymers may generally be between 1,000 and 2 million, preferably between 5,000 and 1 million, more preferably between 10,000 and 0.5 million. Low molecular weight cationic polymers can be preferred. Low molecular weight cationic polymers can have greater translucency in the liquid carrier of a composition.

The cationic guar polymer is a cationically substituted galactomannan (guar) gum derivative. Suitable guar gums for guar gum derivatives may be obtained as a naturally occurring material from the seeds of the guar plant. The cationic guar polymer may be a straight chain mannan which is branched at regular intervals with single membered galactose units on alternative mannose units. The mannose units are linked to each other by means of $\beta$(1-4) glycosidic linkages. The galactose branching arises by way of an $\alpha$(1-6) linkage. Cationic derivatives of the guar gums may be obtained through reactions between the hydroxyl groups of the polygalactomannan and reactive quaternary ammonium compounds. The degree of substitution of the cationic groups onto the guar structure can be sufficient to provide the requisite cationic charge density described above.

The cationic guar polymer may be formed from quaternary ammonium compounds which conform to general Formula I:

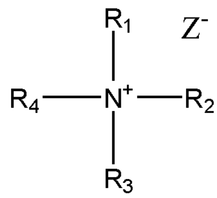

Formula I

wherein where $R^1$, $R^2$ and $R^3$ are methyl or ethyl groups; and $R^4$ is either an epoxyalkyl group of the general Formula II:

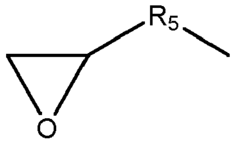

Formula II

or $R^4$ is a halohydrin group of the general Formula III:

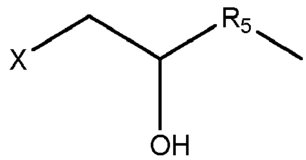

Formula III

wherein $R^5$ is a $C_1$ to $C_3$ alkylene; X is chlorine or bromine, and Z is an anion such as $Cl^-$, $Br^-$, $I^-$ or $HSO_4^-$.

A cationic guar polymer may conform to the general Formula IV:

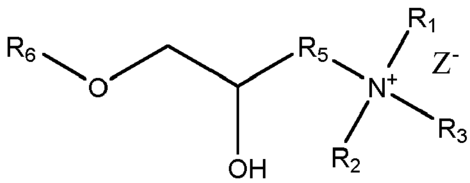

Formula IV

wherein $R^6$ is guar gum; and wherein $R^1$, $R^2$, $R^3$ and $R^5$ are as defined above; and wherein Z is a halogen. A cationic guar polymer may conform to Formula V:

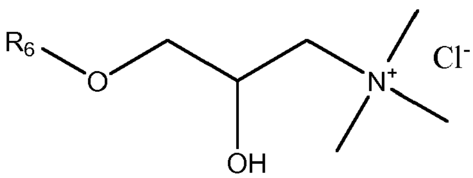

Formula V

wherein $R^6$ is guar gum.

Suitable cationic guar polymers may also include cationic guar gum derivatives, such as guar hydroxypropyltrimonium chloride. Suitable examples of guar hydroxypropyltrimonium chlorides can include the Jaguar® series commercially available from Solvay S.A., Hi-Care™ Series from Rhodia, and N-Hance™ such as N-Hance™ 3000 series, N-Hance™ BF series and N-Hance™ CG series; and AquaCat™ from Ashland Inc. Jaguar® Optima has a charge density of 1.25 meg/g and a M. Wt. of 500,000 g/mol. Jaguar® C-17 has a charge density of 0.8 meq/g and a M.Wt. of 500,000 g/mol; Jaguar® C-500 has a charge density of 0.7 meq/g and a M.Wt. of 500,000 g/mol; Hi-Care™ 1000 has a charge density of 0.7 meq/g and a M.Wt. of 600,000 g/mol; N-Hance™ 3269 and N-Hance™ 3270, have a charge density of 0.7 meq/g and a M.Wt. of 425,000 g/mol; N-Hance™ 3196 has a charge density of 0.6 meq/g and a M.Wt. of 1,000,000 g/mol; N-Hance™ 3296 has a charge density of 0.8 meq/g and a M.Wt. of 1,000,000 g/mol; N-Hance CG-17 has a charge density of 1.0 meq/g and a M.Wt. of 1,000,000 g/mol; AquaCat™ PF618 and AquaCat™ CG518 have a charge density of 0.9 meq/g and a M.Wt. of 50,000 g/mol. N-Hance™ BF-13 and N-Hance™ BF-17 are borate (boron) free guar polymers. N-Hance™ BF-13 has a charge density of 1.1 meq/g and M.W.t of 800,000 g/mol and N-Hance™ BF-17 has a charge

density of 1.7 meq/g and M.W.t of 800,000 g/mol.

## POLYURETHANE MICROCAPSULES

A composition comprises one or more polyurethane microcapsules, preferably a plurality of polyurethane microcapsules. The composition comprises from 0.001% to 10%, preferably from 0.01% to 8%, more preferably from 0.1% to 5%, or even more preferably from 0.25% to 3% of a plurality of polyurethane microcapsules by weight of the composition. The plurality of polyurethane microcapsules comprise a core material and a shell material encapsulating the core material which is disposed within the shell material. The shell material comprises a polyurethane polymer and the core material comprises a benefit agent.

### Shell material

The shell material comprises a polyurethane polymer. The shell material may comprise from 50% to 100%, preferably from 70% to 100%, more preferably from 80% to 100%, by weight of the shell material, of a polyurethane polymer.

The polyurethane polymer is the reaction product of the polymerization between one or more polyisocyanates and glycerine (i.e. 1,2,3-propanetriol).

The one or more polyisocyanates have at least two functional isocyanate groups. The one or more polyisocyanates may have 2, 3 or 4 functional isocyanate groups, preferably 2 or 3 functional isocyanate groups.

The one or more polyisocyanates may comprise a 1,6- or a 1,4-diisocyanate moiety. The one or more polyisocyanates may be derivatives of hexamethylene or of isophrone. The polyisocyanate may be isophrone diisocyanate, hexamethylene diisocyanate, trimer of hexamethylene diisocyanate (Desmodur® N3000, Bayer), trimer of isophrone diisocyanate (Desmodur® Z4470BA, Bayer), or Biuret of hexamethylene diisocyanate (Desmodur® N75BA, Bayer).

Following the above numbers of functional isocyanate groups, a reticulation or network of the shell material is obtained, which can help for providing a prolonged slow release of the benefit agents contained in the core and for providing stability in the composition.

The polyurethane microcapsules may have a mean diameter from 1 $\mu$m to 500 $\mu$m, preferably from 2 $\mu$m to 50 $\mu$m, more preferably from 3 $\mu$m to 30 $\mu$m. "Mean diameter" refers to the arithmetic mean.

The shell material may comprise a polyurea/polyurethane polymer. The polyurea/polyurethane polymer is the reaction product of the polymerization between one or more polyisocyanates and one or more reactants selected from the group consisting of a water-soluble guanidine salt, guanidine and mixture thereof; with glycerine. The guanidine salt may be guanidine carbonate.

Preferred polyurethane microcapsules comprising a shell material comprising a polyurethane polymer material are described in detail in WO 2007/004166 A1.

The polyurethane microcapsules will typically have a volume weighted median particle size from 2 microns to 80 microns, preferably from 3 microns to 60 microns. The volume weighted median particle size of the polyurethane microcapsules may be more preferably from 5 microns to 45 microns, even more preferably from 8 microns to 30 microns. The volume weighted median particle size of the polyurethane microcapsules is determined according to the Volume Weighted Median Particle Size Test Method hereinbelow.

### Core material

The core material disposed within the shell material of the polyurethane microcapsule comprises a benefit agent.

The core material may comprise from 6% to 99.9% of a benefit agent by total weight of the core, preferably from 10% to 90% of a benefit agent by total weight of the core, more preferably from 35% to 85% of a benefit agent by total weight of the core, even more preferably from 60% to 75% of a benefit agent by total weight of the core.

### Benefit agents

Benefit agents useful as core material of the polyurethane microcapsules are generally liquid in form at 25°C. The benefit agent may be preferably a hydrophobic benefit agent such as perfume. Such hydrophobic benefit agents are typically oils.

Suitable benefit agents may include perfumes, brighteners, insect repellants, silicones, waxes, flavors, vitamins, fabric softening agents, skin care agents, enzymes, perfume delivery system; conditioning agents, moisturizers, antibacterial agents, anti-microbial agents, thickeners, sensates, attractants, dyes, pigments, bleaches and mixtures thereof.

The benefit agent may comprise preferably perfumes, brighteners, enzymes, perfume delivery system; conditioning agents, moisturizers, anti-microbial agents, thickeners, sensates, attractants, dyes, pigments, bleaches and

mixtures thereof.

The benefit agent may comprise more preferably one or more perfumes. The one or more perfumes may be selected from any perfume or perfume chemical suitable for topical application to the skin and/or hair and suitable for use in personal care compositions. Alternatively, the one or more perfumes may be selected from any perfume or perfume chemical suitable for topical application to the fabrics and suitable for use in fabric care compositions

The encapsulated perfume can be in the form of a single perfuming ingredient or of an admixture thereof (i.e. a perfuming composition). Said perfuming ingredient may be of synthetic or natural origin. Specific examples of such perfuming ingredient may be found in the current literature, for example in Perfume and Flavour Chemicals, 1969 (and later editions), by S. Arctander, Montclair N.J. (USA). They are well known to the skilled person in the art of perfuming consumer products, that is, of imparting an odour to a consumer product.

Preferably, the perfume may not contain primary alcohols, as these compounds may react with the one or more polyisocyanates during the shell material-formation process. Furthermore, said perfume may contain less than 10% of its own weight of secondary and tertiary alcohols.

Preferably, perfuming ingredients may have 4 to 20 carbon atoms. Perfuming ingredients having 4 to 20 carbon atoms can slowly defuse through the shell material of the polyurethane capsules.

As non-limiting examples of suitable perfuming ingredients one may cite the ones selected from the group of esters, lactones, ketones, ethers or, optionally, the tertiary or secondary alcohols and which are of current use in the perfumery industry. Yet, more particularly one may cite:

- the damascones, such as delta damascone;
- enones, such as 1-(5,5-dimethy1-1-cyclohexen-1-yl)-4-penten-1-one;
- ketones, such as methyl dihydrojasmonate or (1’R)-2-[2-(4’-methyl-3’-cyclohexen-1’-yl)propyl] cyclopentanone;
- esters or lactones, such as 2,2,2-trichloro-1-phenylethyl acetate, methyl dihydrojasmonate or pentadecenolide; or
- ethers, such as dodecahydro-3a,6,6,9a-tetramethyl-naphtho[2,1-b]furan.

The polyurethane microcapsule may contain from 60 % to 98 % of perfume, relative to their total weight. Alternatively, the polyurethane microcapsule may contain from 85 % to 95 % of perfume relative to their total weight.

Process of making polyurethane or polyurethane/polyurea microcapsules

The polyurethane or polyurethane/polyurea microcapsules may be preferably prepared, in a first step, by preparing an emulsion or dispersion comprising a hydrophobic solution, a water phase, the one or more polyisocyanates with glycerine or respectively the one or more polyisocyanates and the one or more reactants selected from the group consisting of a water-soluble guanidine salt, guanidine and mixture thereof with glycerine, and wherein the droplet size is comprised between above 1 μm and 500 μm.

Preferably, the hydrophobic solution may be prepared by mixing the perfume with hydrophobic solvents of current use in the perfume industry, which are preferably not alcohols. Examples of such solvents may be diethyl phthalate, isopropyl myristate, benzyl benzoate, ethyl citrate, limonene or other terpenes, or isoparaffins.

Preferably, the hydrophobic solution may comprise less than 30 wt.% of solvent. More preferably, the hydrophobic solution comprises less than 20 wt.%, and even more preferably less than 10 wt.% of solvent. Most preferably, the hydrophobic solution may consist essentially of perfume and is essentially free of a solvent.

Preferably, the emulsion or dispersion comprises 10-50 wt.% of perfume, more preferably 20-40 wt.% of perfume.

The one or more polyisocyanates may then be added to the hydrophobic solution. Preferably, the one or more polyisocyanates may be added at a percentage from 2 to 20 wt.% of the hydrophobic solution

The emulsion or dispersion may be prepared by high shear mixing and adjusted to the desired droplet size. Droplet size may be checked with light scattering measurements or microscopy. An emulsion may be characterized by the stabilization of the oil droplets by emulsifiers, while in a dispersion the droplets are generally stabilized by a colloidal stabilizer. Accordingly, an emulsifier and/or a colloidal stabilizer may be preferably added to the emulsion or dispersion. Examples of colloidal stabilizers may be polyvinyl alcohol, cellulose derivatives such hydroxyethyl cellulose, polyethylene oxide, copolymers of polyethylene oxide and polyethylene or polypropylene oxide, or copolymers of acrylamide and acrylic acid.

Examples of emulsifier are surfactants such as stepantex®, non-ionic surfactant such as diblock copolymers of polyethylene oxide and polyethylene or polypropylene oxide.

At this stage, the one or more reactants if needed (e.g. guanidine carbonate) may be added, preferably after mixing with water. Preferably, for each mole of isocyanate present in the hydrophobic solution 1 to 3, preferably 1.2 to 2 moles of primary amine groups (in reactant) are added to the emulsion. Accordingly, the emulsion or dispersion may preferably comprise an excess of reactant.

In a further step, a polymerization between the one or more polyisocyanates and the one or more reactants in the emulsion or dispersion may be induced. In the case of guanidine or its salts, no specific action is required for inducing the polymerization, because the reaction may start immediately after adding the polyamines to the aqueous solution.

In the case of mixture comprising glycerine, the polymerization may be helped by slowly heating, preferably from 40°C to 90°C in from 0.5 to 5 hours. In addition or alternatively, polymerization may be induced by addition of a catalyst. An example for a suitable catalyst is 1,4-diazabicyclo[2.2.2]octane. Preferably, the reaction may be maintained for 2 to 30, preferably 5 to 20 hours.

## COACERVATE

The composition comprises one or more coacervates formed with the anionic surfactant system, preferably the anionic surfactant of the anionic surfactant system, and the cationic guar polymer.

A physicochemical complex is formed between the sodium laureth(n) sulfate and the cationic guar polymer within the composition typically upon water dilution of the composition. Coacervate formation is dependent upon a variety of factors, such as polymer molecular weight, component concentration, ratio of components, ionic strength, charge density, the types of surfactants, the pH of the composition and the temperature of the composition. Coacervate systems and the effect of these parameters have been described, for example, in J. Caelles et al., Anionic and Cationic Compounds in Mixed Systems, 106 Cosmetics & Toiletries 49, 49-54 (April 1991), C. J. van Oss, Coacervation, Complex-Coacervation and Flocculation, 9 J. Dispersion Science and Tech., 561, 561-573, (1988-89), and in D. J. Burgess, Practical Analysis of Complex Coacervate Systems, 140 (1) J. of Colloid and Interface Science, 227, 227-238, (November 1990).

It has been surprisingly found that the nature of the cationic polymer being a cationic guar polymer and the charge density of the cationic guar polymer being from 0.6 meq/g to 2.5 meq/g affect the characteristics of the obtained one or more coacervates.

As shown in the Experimental part, the one or more coacervates formed between the anionic surfactant sodium laureth(n) sulfate and the cationic guar polymer having a charge density between 0.6 meq/g to 2.5 meq/g can lead to a relatively large product agglomeration with a significant number of spherical clusters of polyurethane microcapsules associated with the relatively large mass agglomerates. Hence, a cluster formation of the polyurethane microcapsules within the one or more coacervates can been obtained.

It is believed that the polyurethane microcapsules can readily aggregate as clusters to be included in the one or more coacervates, compared to other types of polymer microcapsules. In that case, the experimental part has also shown that only one type of a cationic guar polymer having a charge density from 0.6 meq/g to 2.5 meq/g is sufficient to form the one or more coacervates with the anionic surfactant of the anionic surfactant system satisfactorily. For instance, Ex. 18 has shown that the use of a cationic guar polymer with a second polymer such as Acrylates/C10-30 Alkyl Acrylate Crosspolymer did not provide an increased formation of the one or more coacervates compared using only a cationic guar polymer.

The one or more coacervates of the composition has an enhanced viscoelasticity. This increase indicated a strong polyelectrolyte interaction, namely a coacervate, between the cationic guar polymer and the anionic surfactant system, here SLES/SLS.

The viscoelasticity of isolated coacervates has been assessed according to the Viscosity Test Method and the Tan Delta Test Method as disclosed herein.

The one or more coacervates of the composition have a relatively enhanced viscosity compared to the remaining of the composition. In other words, the isolated coacervates are much thicker than the remaining of the composition. The one or more coacervates of the composition have a relatively lower tan $\delta$ means than the remaining of the composition. Namely, the isolated coacervates are much more elastic (or solid-like) than the remaining of the composition. These properties characterize the formation of the one or more coacervates. These properties can be translated into more deposition of the one or more coacervates of the composition onto the surface of interest.

The deposition index of the composition comprising the one or more coacervates according to the charge density of the cationic guar polymer was determined.

The deposition index is above or equal to 1.0 when the cationic guar polymer has a charge density comprised between 0.6 meq/g to 2.5 meq/g. Preferably, the deposition index is above or equal to 1.5 when the cationic guar polymer has a charge density comprised between from 0.75 meq/g to 2.0 meq/g. More preferably, the deposition index is above or equal to 2.0 when the cationic guar polymer has a charge density comprised between from 1.0 meq/g to 1.75 meq/g.

The one or more coacervates comprise one or more clusters, wherein the one or more clusters includes a portion of the plurality of polyurethane microcapsules.

It has been surprisingly found that the development of clusters of polyurethane microcapsules included within the one or more coacervates can help for the deposition of polyurethane microcapsules delivered to a situs via the composition. In this regard, the formation of clusters of polyurethane microcapsules within the one or more coacervates of the composition containing polyurethane microcapsules can improve the deposition of the polyurethane microcapsules

onto the surface of interest, and thereby can lead to a more noticeable bloom of benefit agents, e.g. fragrances from the plurality of polyurethane microcapsules.

Indeed, it has been shown below that the one or more coacervates formed between the anionic surfactant sodium laureth(n) sulfate and the cationic guar polymer having a charge density between 0.6 meq/g to 2.5 meq/g can lead to a relatively large product agglomeration with a significant number of spherical clusters of polyurethane microcapsules associated with the relatively large mass agglomerates. Hence, a cluster formation of the polyurethane microcapsules within the one or more coacervates can been obtained.

The more the polyurethane microcapsules form clusters associated with the one or more coacervates of the composition, the more the deposition of the polyurethane microcapsules is obtained onto the surface of interest, e.g. the skin.

The formation of clusters of polyurethane microcapsules within the one or more coacervates of the composition containing polyurethane microcapsules can help to provide a noticeable fragrance longevity benefit up to 4 hours or longer compared to the same product without the cationic guar-type polymer or with a different cationic guar polymer of inadequate polymer charge density.

The cluster formation of the polyurethane microcapsules within the one or more coacervates of the composition can explain the relatively acceptable fragrance longevity benefit up to 4 hours or longer.

Hence, the one or more coacervates may preferably comprise a plurality of clusters, wherein the plurality of clusters includes a portion of the plurality of polyurethane microcapsules. The portion of the plurality of polyurethane microcapsules may range from 5 to 50, preferably from 10 to 40, more preferably from 15 to 35 polyurethane microcapsules per coacervate, as based on a visible assessment, i.e. counting, on a light micrograph, generated by a light microscope Zeiss AX10 Imager.A2 with 40X magnification from Carl Zeiss Co.; Goettengen, Germany.

Having one or more coacervates comprising a plurality of clusters which includes a portion of the plurality of polyurethane microcapsules, a fragrance longevity benefit can be obtained, namely that a fragrance originating from the plurality of polyurethane microcapsules deposited onto the skin is noticed up to 4 hours or longer after using the composition.

## Liquid Carrier

Inclusion of an appropriate quantity of a liquid carrier can facilitate the formation of the composition having an appropriate liquid viscosity and rheology. The composition may include, by weight of the composition, from 50% to 95%, of a liquid carrier, preferably from 60% to 85%, more preferably from 65% to 80%, even more preferably from 68% to 78%, and/or even further more preferably from 70% to 77%.

A liquid carrier may be water, or may be a miscible mixture of water and organic solvent. Alternatively, a liquid carrier may be water with minimal or no significant concentrations of organic solvent, except as otherwise incidentally incorporated into the composition as minor ingredients of other essential or optional components. Suitable organic solvents may include water solutions of lower alkyl alcohols and polyhydric alcohols. Useful lower alkyl alcohols include monohydric alcohols having 1 to 6 carbons, such as ethanol and isopropanol. Exemplary polyhydric alcohols include propylene glycol, hexylene glycol, glycerin, and propane diol.

## Optional ingredients

As can be appreciated, the compositions described herein may include a variety of optional components to tailor the properties and characteristics of the composition. As can be appreciated, suitable optional components are well known and can generally include any components which are physically and chemically compatible with the essential components of the compositions described herein. Optional components should not otherwise unduly impair product stability, aesthetics, or performance. Individual concentrations of optional components can generally range from 0.001% to 10%, by weight of the composition. Optional components can be further limited to components which will not impair the clarity of a translucent composition.

Optional components may include, but are not limited to, conditioning agents (including hydrocarbon oils, fatty esters, silicones), cationic polymers, anti-dandruff actives, and chelating agents. Additional suitable optional ingredients include but are not limited to non-encapsulated perfumes or fragrances, colorants, particles, anti-microbials, foam boosters, anti-static agents, propellants, self-foaming agents, pH adjusting agents and buffers, preservatives, pearlescent agents, solvents, diluents, anti-oxidants, vitamins and combinations thereof.

Such optional ingredients should be physically and chemically compatible with the components of the composition, and should not otherwise unduly impair product stability, aesthetics, or performance. The CTFA Cosmetic Ingredient Handbook, Tenth Edition (published by the Cosmetic, Toiletry, and Fragrance Association, Inc., Washington, D.C.) (2004) (hereinafter "CTFA"), describes a wide variety of nonlimiting materials that can be added to the composition herein.

## FORMS AND USES

### Product Form

The composition may be presented in typical hair care, personal care or fabric care formulations. They may be in the form of solutions, dispersion, emulsions, foams, and other delivery mechanisms. The composition may be a micellar composition.

The composition may be liquid or solid. The composition may be the form of a hair shampoo, a hair conditioner, a liquid detergent, a fabric softener, a shower or bath cream, a body wash or foaming body wash, gel, emulsion, oil, mousse or spray, a deodorant on an antiperspirant consumer product.

The composition in the form of a foam may have a density of from 0.02 $g/cm^3$ to 0.2 $g/cm^3$, alternatively from 0.025 $g/cm^3$ to 0.15 $g/cm^3$, alternatively from 0.05 $g/cm^3$ to 0.15 $g/cm^3$. The density can be measured Foam Density & Foam Volume Method, described hereafter.

### Foam Dispenser

The composition may be stored and dispensed from a mechanical pump foam dispenser that may comprise a reservoir for holding the composition and a foam engine. The reservoir may be made from any suitable material selected from the group consisting of plastic, metal, alloy, laminate, and combinations thereof. The reservoir may be for one-time use. The reservoir may be removable from the mechanical pump foam dispenser. Alternatively, the reservoir may be integrated with the mechanical pump foam dispenser. There may be two or more reservoirs.

The reservoir may be comprised of a material selected from the group consisting of rigid materials, flexible materials, and combinations thereof. The reservoir can be made from plastic.

Suitable foam dispenser available from Albéa can include tabletop models T1, WRT4, WRT6, and handheld models M3, WRM3, WRD4, F2, F3 and G3 with a L value of 7, 9 or 11, wherein L value is the air to liquid ratio.

The composition may be stored and dispensed from a squeeze foam dispenser. An example of squeeze foamer is EZ'R available from Albéa.

Non-limiting examples of suitable pump dispensers include those described in WO 2004/078903, WO 2004/078901, and WO 2005/078063 and may be supplied by Albea (60 Electric Ave., Thomaston, CT 06787 USA) or Rieke Packaging Systems (500 West Seventh St., Auburn, Indiana 46706).

The composition and/or the dispenser may be free or substantially free of a propellant, for instance aerosol propellants.

### Uses

An aspect of the present invention is related to the use of a composition comprising:

(a) an anionic surfactant system comprising from 1% to 30% of an anionic surfactant by total weight of the composition, wherein the anionic surfactant is sodium laureth(n) sulfate SLEnS, wherein n is the average moles of ethoxylation, wherein n ranges from 0 to 8, preferably from 1 to 3;
(b) from 0.01% to 2% of a cationic guar polymer by total weight of the composition, wherein the cationic guar polymer has a charge density from 0.6 meq/g to 2.5 meq/g;
(c) one or more coacervates formed with the anionic surfactant system, preferably with the anionic surfactant, and the cationic guar polymer; and
(d) from 0.001% to 10% of a plurality of polyurethane microcapsules by weight of the composition, wherein the polyurethane microcapsules comprise a shell material encapsulating a core material, said core material being disposed within said shell material, wherein said shell material comprises a polyurethane polymer and said core material comprises a benefit agent, wherein the polyurethane polymer of the shell material is the reaction product of the polymerization between one or more polyisocyanates and glycerine, and wherein the one or more polyisocyanates have at least two functional isocyanate groups,

wherein the one or more coacervates comprise one or more clusters, wherein the one or more clusters includes a portion of the plurality of polyurethane microcapsules;
for providing a long-lasting benefit on a surface of interest, preferably hair, skin or fabrics, more preferably hair or skin.

The composition may be used for noticing a fragrance benefit onto the surface of interest, i.e. hair, skin or fabrics, preferably skin for at least 1 hour after initial use. Alternatively, the composition may be used for noticing a fragrance benefit onto the surface of interest, i.e. hair, skin or fabrics, preferably skin, up to 4 hours or longer, i.e. even after 8 hours after initial use. Preferably, the composition may be used for noticing a fragrance benefit onto the surface

of interest, i.e. hair, skin or fabrics, preferably hair or skin, more preferably skin from 1 hour to 4 hours after initial use.

Also, the composition may be used for improving the viscoelasticity of the one or more coacervates in a micellar anionic surfactant system.

## TEST METHODS

It is understood that the test methods that are disclosed in the Test Methods Section of the present application should be used to determine the respective values of the parameters of Applicants' invention as such invention is described and claimed herein.

### VISCOSITY TEST METHOD

The viscosity of the composition is measured using a Brookfield Viscometer (model RVDVII) with a CPE-41 Spindle with temperature control. The system parameters are listed below:

| | |
|---|---|
| Temperature: | 25°C |
| Spindle Rotation Speed: | 1 RPM (2 $s^{-1}$) |
| Gap: | 13 micron |
| Duration: | 2 minutes |
| Cone Angle: | 3° |

### VOLUME WEIGHTED MEDIAN PARTICLE SIZE

Particle size is measured using an Accusizer 780A, made by Particle Sizing Systems, Santa Barbara CA. The instrument is calibrated from 0 to 300 $\mu$m using Duke particle size standards. Samples for particle size evaluation are prepared by diluting 1 g emulsion, if the volume weighted median particle size of the emulsion is to be determined, or 1 g of microcapsule slurry, if the finished capsule volume weighted median particle size is to be determined, in 5 g of de-ionized water and further diluting 1 g of this solution in 25 g of water. 1 g of the most dilute sample is added to the Accusizer and the testing initiated, using the autodilution feature. The Accusizer should be reading in excess of 9200 counts/second. If the counts are less than 9200 additional sample should be added. The Accusizer will dilute the test sample until 9200 counts/second and initiate the evaluation. After 2 min of testing the Accusizer will display the results, including volume-weighted median size.

The broadness index can be calculated by determining the particle size at which 95% of the cumulative particle volume is exceeded (95% size), the particle size at which 5% of the cumulative particle volume is exceeded (5% size), and the median volume-weighted particle size (50% size-50% of the particle volume both above and below this size).

$$\text{Broadness Index (5)} = ((95\%\ \text{size})\text{-}(5\%\ \text{size})/50\%\ \text{size}).$$

### TAN DELTA TEST METHOD determined by Oscillatory Rheology Tests

To measure the viscous (G") and elastic (G') moduli (loss and storage modulus) of a composition, a AR G2 Rheometer (TA Instruments, DE, USA) is used and is equipped with 1 degree cone upper geometry with a diameter of 40 mm and a flat plate lower geometry equipped with Pletier heating/cooling to control temperature. Measurement can be conducted by placing approximately 1 gram of the composition onto a lower test geometry and lowering the upper geometry to the desired gap of 27 microns, wiping away any excess of the composition to create an even surface around the edge of the geometry. The oscillatory test is conducted over frequency range of 0.01 to 100 radians per second, collecting 10 data points per decade, using a constant oscillatory stress of 0.1 Pa utilizing and a set temperature of 25°C. Oscillation stress sweeps are run to generate the elastic modulus or storage modulus (G'), the viscous modulus or loss modulus (G") and Tan Delta which is calculated as the follows:

$$\tan\delta = \frac{G''}{G'}$$

FOAM DENSITY AND FOAM VOLUME

Foam density is measured by placing a 100 mL beaker onto a mass balance, tarring the mass of the beaker and then dispensing product from the aerosol container into the 100 mL beaker until the volume of the foam is above the rim of the vessel. The foam is made level with the top of the beaker by scraping a spatula across it within 10 seconds of dispensing the foam above the rim of the vessel. The resulting mass of the 100 mL of foam is then divided by the volume (100) to determine the foam density in units of g/mL.

Foam volume is measured by placing a weigh boat onto a mass balance, tarring the mass of the weigh boat and then dispensing the desired amount of product from the aerosol container. The grams of foam dispensed is determined and then divided by the density of foam as determined from the Foam Density methodology to reach a volume of foam in mL or $cm^3$.

**EXAMPLES**

The following examples further describe and demonstrate embodiments within the scope of the present invention. The examples are given solely for the purpose of illustration and are not to be construed as limitations of the present invention, as many variations thereof are possible without departing from the spirit and scope of the invention. Where applicable, ingredients are identified by chemical or CTFA name, or otherwise defined below.

Example PU1 of a polyurethane microcapsule according to the present invention Encapsulation of a single perfuming ingredient in the polyurethane Capsules

Synthesis of polyurethane capsules:

| Chemicals | Amounts |
|---|---|
| Desmodur N3300(1) | 2.10 g |
| Perfume (a-Damascone)(2) | 30.00 g |
| Propantriol | 0.27 g |
| Celvol 523(3), 1 % in water | 70.00 g |
| Deionised water | 4.77 g |
| DABCO(4) | 0.02 g |
| Total | 107.16 g |

(1) polyisocyanate, Bayer
(2) (+-)-(E)-1-(2,6,6-trimethyl-2-cyclohexen-1-yl)-2-buten-1-one
(3) polyvinyl alcohol, Celanese Chemicals
(4) 1,4-Diazabicyclo[2.2.2]octane, Fluka

The stabilizer solution (water + Celvol) with the oil phase (hydrophobic solution comprising the perfume and the polyisocyanate) is stirred for 1 minute on magnetic stirrer. The oil phase is then emulsified by ultra-turrax for 4 minutes when the temperature is maintained at 25°C. The size distribution is controlled by light scattering measurements and revealed that the mean diameter of oil droplets is 9 μm. The emulsion is transferred to the reactor. Propantriol is mixed with water and added gradually to the emulsion (during 3 min). The suspension is then heated from 25°C to 70°C during 2 hours. The reaction mixture is kept at 70°C for 16 hours. At the end of the polymerization process, the reaction mixture is cooled down. The resulting capsules suspension is then characterized by determining its solid content and size distribution. The solid content is determined to be 32.4 wt.% (including the perfume; perfume contents of the capsules: 85-88%) by heating 1 g of the final capsule suspension at 50°C for a duration comprising between 3 to 7 hours, mean size of capsules suspension was 9 μm.

The following examples were prepared:

Compositions (wt.%)

| Ingredients | Ex.1 | Ex. 2 | Ex. 3 | Ex. 4 | Ex. 5 | Ex. 6 | Ex. 7 | Ex. 8 |
|---|---|---|---|---|---|---|---|---|
| Water | 59.84 | 61.22 | 58.04 | 59.84 | 61.22 | 59.84 | 61.22 | 58.04 |
| SLES (28% active)[1] | 23.20 | 25.00 | 23.20 | 23.20 | 25.00 | 23.20 | 25.00 | 23.20 |

(continued)

| Ingredients | Ex.1 | Ex. 2 | Ex. 3 | Ex. 4 | Ex. 5 | Ex. 6 | Ex. 7 | Ex. 8 |
|---|---|---|---|---|---|---|---|---|
| CAPB (30% active)[2] | 3.33 | 10.00 | 3.33 | 3.33 | 10.00 | 3.33 | 10.00 | 3.33 |
| SLS (29% active)[3] | 8.60 | - | 8.60 | 8.60 | - | 8.60 | - | 8.60 |
| Trihydroxystearin[4] | 0.50 | 0.25 | 0.50 | 0.50 | 0.25 | 0.50 | 0.25 | 0.50 |
| Sodium Benzoate[5] | 0.25 | 0.25 | 0.25 | 0.25 | 0.25 | 0.25 | 0.25 | 0.25 |
| Methylchloroisothiazolinone/ Methylisothiazo linone (MCIT/MIT)[6] | 0.03 | 0.03 | 0.03 | 0.03 | 0.03 | 0.03 | 0.03 | 0.03 |
| Fragrance | 1.00 | 1.00 | 2.00 | 1.00 | 1.00 | 1.00 | 1.00 | 2.00 |
| Disodium EDTA[7] | 0.10 | 0.10 | 0.10 | 0.10 | 0.10 | 0.10 | 0.10 | 0.10 |
| Citric Acid[8] | 0.15 | 0.15 | 0.15 | 0.15 | 0.15 | 0.15 | 0.15 | 0.15 |
| NaCl[9] | 2.00 | 1.00 | 2.00 | 2.00 | 1.00 | 2.00 | 1.00 | 2.00 |
| Guar Hydroxypropyltrimonium Chloride[10] | 0.20 | 0.20 | 0.20 | - | - | - | - | - |
| Guar Hydroxypropyltrimonium Chloride[11] | - | - | - | 0.20 | 0.20 | - | - | - |
| Guar Hydroxypropyltrimonium Chloride [12] | - | - | - | - | - | 0.20 | 0.20 | 0.20 |
| Polyurethane microcapsules (25% perfume)[13] | 0.80 | 0.80 | 1.60 | 0.80 | 0.80 | 0.80 | 0.80 | 1.60 |

Definitions of Components

*1 Sodium Laureth Sulfate; Supplier Procter & Gamble Co.
*2 Cocamidopropyl Betaine; Supplier BASF
*3 Sodium Lauryl Sulfate; Supplier Procter & Gamble Co.
*4 Thixcin R; Supplier Elementis Specialties Co.
*5 Purox S Grains; Supplier Emerald Kalama Chemical
*6 Kathon; Supplier Rohm & Haas
*7 Disodium Ethylene Diamine Tetraacetic Acid; Supplier Akzo Nobel
*8 Citric Acid; Supplier Cargill
*9 Sodium Chloride; Supplier Morton International
*10 N-Hance 3270; Supplier Hercules
*11 N-Hance 3196; Supplier Hercules
*12 Jaguar C500; Supplier Rhodia
*13 Polyurethane microcapsules; Supplier Firmenich under the tradename PopScent®

Method of Preparation

The above compositions of "Ex. 1" through "Ex. 8" were prepared by the following method according to the proportions provided in the Table hereinabove:

Water was first added to a container along with a magnetic stir bar. Sodium Laureth Sulfate, Cocamidopropyl Betaine, and Sodium Lauryl Sulfate when applicable were added. The system was mixed at 300-400 rpm for 2 minutes. Trihydroxystearin was added and the system mixed for an additional 2 minutes at 300-400 rpm. Sodium benzoate, Methylchloroisothiazolinone / Methylisothiazolinone, fragrance, EDTA, and citric acid were added and the system mixed for an additional 2 minutes at 300-400 rpm. Then, sodium chloride was added and the system mixed until thickened; 1 minute at 300-400 rpm, Thereafter, the corresponding Guar Hydroxypropyltrimonium Chloride and Polyurethane microcapsules were added. The system was mixed slowly at 100-200 rpm for 2 minutes until the system was homogeneous.

**EXPERIMENTAL**

COACERVATE FORMATION ASSESSMENT

The following compositions of "Ex. 11" through "Ex. 23" were prepared according to the general composition of Ex. 1 comprising 0.2 wt.% of a cationic polymer to be tested as indicated in the Table below. "Ex. 11" through "Ex. 23" differ from each other by the type of cationic polymer. The following compositions of "Ex. 11" through "Ex. 23" have been prepared according to the Method of Preparation just described above.

Compositions (wt.%)

| Ingredients | Ex. 1 |
|---|---|
| Water | 59.84 |
| SLES (28% active)[1] | 23.20 |
| CAPB (30% active)[2] | 3.33 |
| SLS (29% active)[3] | 8.60 |
| Trihydroxystearin[4] | 0.50 |
| Sodium Benzoate[5] | 0.25 |
| Methylchloroisothiazolinone/ Methylisothiazolinone (MCIT/MIT)[6] | 0.03 |
| Fragrance | 1.00 |
| Disodium EDTA[7] | 0.10 |
| Citric Acid[8] | 0.15 |
| NaCl[9] | 2.00 |
| ***Cationic Polymer to be tested (see Exs. 11-23)** | 0.20 |
| Polyurethane microcapsules (25% perfume)[13] | 0.80 |

| Cationic Polymer to be assessed | Ex. | Charge density (meq/g) | Coacervate formation |
|---|---|---|---|
| Nonionic Polymer, Polyethylene Polyethylene Exxon MW 1,000,000 Daltons (control) | 11 | 0 | None |
| Guar Hydroxypropyltrimonium Chloride N-Hance 3196 (Hercules Inc., Aqualon Division) | 12 | 0.6 | Average |
| Polyquaternium 10, a quaternized hydroxyethyl cellulose UCARE JR400 (Dow Chemical) | 13 | 0.7 | Poor |
| Guar Hydroxypropyltrimonium Chloride Jaguar C500 (Rhodia) MW circa 500,000 Daltons | 14 | 0.7 | Average |
| Guar Hydroxypropyltrimonium Chloride Jaguar C17 (Rhodia) MW ~500,000 Daltons | 15 | 0.8 | Average |
| Guar Hydroxypropyltrimonium Chloride N-Hance 3296 (Hercules Inc., Aqualon Division) | 16 | 0.8 | Average |
| Guar Hydroxypropyltrimonium Chloride N-Hance CG-17 Cationic Guar (Hercules Inc., Aqualon Division) | 17 | 1.0 | Good |
| Guar Hydroxypropyltrimonium Chloride N-Hance CG-17 + Aqupec Ser W-300C (Acrylates/C10-30 Alkyl Acrylate Crosspolymer, Sumitomo Seika Chemicals) MW 1,000,000 Daltons | 18 | 1.0/2.0 | Poor |

(continued)

| Cationic Polymer to be assessed | Ex. | Charge density (meq/g) | Coacervate formation |
|---|---|---|---|
| Copolymer of acrylamide and triquaternary, MW=1,000,000 Daltons Mirapol AM-Triquat or Polyquaternium 76 (Rhodia) | 19 | 1.6 | Poor |
| Copolymer of acrylamide and diallyldimethylammonium chloride Polyquaternium-7 (BASF) | 20 | 1.6 | Poor |
| Acrylates/C10-30 Alkyl Acrylate Crosspolymer Aqupec Ser W-300C (Sumitomo Seika Chemicals) | 21 | 2.0 | None |
| Copolymer of polydiallydimethylammonium chloride (DADMAC) Merquat 100 (Nalco) | 22 | 2.0 | Poor |
| Polyethyleneimine Lupasol SK (BASF)[7] MW ~ 2,000,000 Daltons | 23 | 8.0 | None |
| "Good" means that one or more coacervates have been present within light microscopy assessment, with many polyurethane microcapsules were found within the one or more coacervates (meaning a significant cluster formation), so that significant perfume longevity results.<br>"Average" means that one or more coacervates may have been present within light microscopy assessment, but only a few polyurethane microcapsules were found within the one or more coacervates (meaning some cluster formation), so that limited perfume longevity results.<br>"Poor" means that one or more coacervates may be formed, but virtually no polyurethane microcapsules were found within the one or more coacervates.<br>"None" means that no coacervate was formed. | | | |

The coacervate formation was assessed via a light microscope (Zeiss AX10 Imager.A2 with 40X magnification from Carl Zeiss Co.; Goettengen, Germany). Three micrographs corresponding to Examples 13, 18 and 17 have been generated (See Fig. 1).

The micrograph 1 of Ex. 13 comprising PQ-10 as the cationic polymer showed some coacervates 1 with no polyurethane microcapsules associated with the one or more coacervates. This resulted in poor coacervate formation and no fragrance longevity benefit has been observed.

The micrograph 2 of Ex. 18 comprising a combination of cationic guar polymers of N-Hance CG-17 + Aqupec Ser W-300C showed the formation of some coacervates 1 including few spherical clusters 2 which represent an agglomerate of a plurality of polyurethane microcapsules associated with the one or more coacervates. The combination of cationic guar polymer with Acrylates/C10-30 Alkyl Acrylate Crosspolymer resulted in poor coacervated formation and no fragrance longevity benefit has been observed.

The micrograph 3 of Ex. 17 comprising Guar Hydroxypropyltrimonium Chloride N-Hance CG-17 showed a relatively large product agglomeration with a significant number of spherical clusters 2 associated with the relatively large mass agglomerates. The relatively large product agglomeration 1 is the one or more coacervates of the composition; the spherical clusters 2 are the agglomerate of the plurality of the polyurethane microcapsules associated with the one or more coacervates. Hence, a cluster formation of the polyurethane microcapsules within the one or more coacervates has been obtained. It is alleged that the polyurethane microcapsules can readily aggregate as clusters to be inserted in the coacervates, compared to other types of polymer microcapsules.

Furthermore, the viscoelasticity of Ex. 17 has been measured in comparison to Ex. 10 comprising no cationic polymer. The presence of 0.2 wt.% guar Hydroxypropyltrimonium Chloride with a charge density of 1.0 meq/g has resulted in a significant increase in viscoelasticity. This increase indicated a strong polyelectrolyte interaction, namely a coacervate, between the cationic guar polymer and the anionic surfactant system, here SLES/SLS.

The viscoelasticity of isolated coacervates from Ex. 17 has been assessed according to the Viscosity Test Method and the Tan Delta Test Method as disclosed herein.

A control reference has been prepared by taking the same Ex. 17 without any cationic guar polymer but still with a plurality of polyurethane microcapsules. The control reference showed a viscosity of 10,000 cps at 25°C under a shear rate of 2 $s^{-1}$, and a tan $\delta$ = 0.4.

The one or more coacervates of Ex. 17 were isolated. The remaining solution of Ex. 17 has shown a similar viscosity of 10,000 cps at 25°C under a shear rate of 2 $s^{-1}$, and a tan $\delta$ = 0.4. However, the isolated coacervates of Ex. 17 have shown a viscosity above 100,000 cps at 25°C under a shear rate of 2 $s^{-1}$, and a tan $\delta$ = 0.1.

An increase in viscosity indicated that the isolated coacervates are much thicker than the remaining of the

composition. A lower tan δ means that the isolated coacervates are much more elastic (or solid-like) than the remaining of the composition. These properties characterize the formation of the one or more coacervates. These properties can be translated into more deposition of the one or more coacervates of the composition onto the surface of interest, e.g. skin as shown more in detail below.

The deposition index of the composition comprising the one or more coacervates according to the charge density of the cationic guar polymer was also determined.

All product tested below used the composition of Ex. 1 as set out above with 0.2 wt.% of the selected cationic guar polymer and a fixed amount of 0.8 wt.% polyurethane microcapsules. The other ingredients in Ex. 1 and the Method of Preparation remain the same.

The amount of spherical clusters formed within the one or more coacervates for each example was compared to the ones obtained when using as the cationic polymer N-Hance 3196. The spherical clusters of the plurality of the polyurethane microcapsules in each coacervate were counted and averaged, i.e. how many spherical clusters 2 were found within a coacervate 1. The value of the deposition index is 1 at a charge density of 0.6 meq/g for Ex. 12 comprising N-Hance 3196. The cationic guar polymer N-Hance 3196 has been used as a reference for the coacervated formation.

The deposition index has been obtained by a visual account of the average number of polyurethane microcapsules associated within each coacervate via Light Microscopy:

Deposition Index = (Amount of polyurethane microcapsules associated within each coacervate for a given cationic guar polymer) / (Amount of polyurethane microcapsules associated within each coacervate for N-Hance 3196)

**Table 1: Deposition Index versus the Charge Density of the cationic guar polymer**

| Ex. | Cationic guar polymer | Charge density (meq/g) | Numbers of clusters formed and observed by Light Microscopy | Deposition Index |
|---|---|---|---|---|
| 11 | Polyethylene | 0.0 | 0 | 0.0 |
| 12 | N-Hance 3196[11] | 0.6 | 20 | 1.0 |
| 14 | Jaguar 500[12] | 0.7 | 25 | 1.25 |
| 15 | N-Hance CG-17[10] | 1.0 | 42 | 2.10 |
| 15b | N-Hance CG-17[10] | 1.0 | 43 | 2.15 |
| 15c | N-Hance CG-17[10] | 1.0 | 45 | 2.25 |
| 24 | N-Hance CG-17 with increased charge[14] | 1.7 | 42 | 2.10 |
| 25 | N-Hance CG-17 with increased charge[14] | 2.5 | 20 | 1.0 |
| *10 N-Hance CG-17; Supplier Hercules<br>*11 N-Hance 3196; Supplier Hercules<br>*12 Jaguar C500; Supplier Rhodia<br>*14 N-Hance CG-17; Supplier Rhodia with added charged units | | | | |

Fig. 2 shows the deposition index versus the charge density of the cationic guar polymer. The deposition index is above or equal to 1.0 when the cationic guar polymer has a charge density comprised between 0.6 meq/g to 2.5 meq/g. Preferably, the deposition index is above or equal to 1.5 when the cationic guar polymer has a charge density comprised between from 0.75 meq/g to 2.0 meq/g. More preferably, the deposition index is above or equal to 2.0 when the cationic guar polymer has a charge density comprised between from 1.0 meq/g to 1.75 meq/g.

The more the polyurethane microcapsules form clusters associated with the one or more coacervates of the composition, the more the deposition of the polyurethane microcapsules is obtained onto the surface of interest, e.g. skin.

FRAGRANCE LONGEVITY SENSORY ASSESSMENT

Fragrance longevity benefit can be defined by having a noticeable fragrance lasting more than 1 hour. Sensory

tests were conducted in order to assess a fragrance longevity benefit. Sensory testing was conducted to show the fragrance longevity benefit of the deposition of the plurality of the polyurethane microcapsules onto the skin.

Information about the panel of the testers:

A first series of sensory Tests were conducted by testing the examples below among 10 or 12 individuals per test. Each test has been carried out in a blind manner. Each P&G employee was not biased. Indeed, no tester has received any prior information about the sample to be tested and any information about the project.

Information about the conduct of the test:

10 grams of each product (Control, Ex. 13, Ex. 17 and Ex. 18) contained within a small plastic, capped syringe was provided to each panelist. Each panelist used the products at home under normal shower wash conditions/habits. Each panelist was asked to note after each hour during the day after initial use if he noticed any fragrance or not. Panelists were requested to note fragrance each hour up to 8 hours after initial use. The limit of greater than 1 hour was considered "acceptable." Shorter than a 1 hour benefit was deemed to be "unacceptable."

**Table 2 Fragrance longevity sensory results**

| Sample to be tested | Fragrance Longevity Benefit |
|---|---|
| Control, Ex. 1: no cationic polymer | Poor, Initial exposure only (time = 0-1 hours) 1 out of 10 testers noticed the fragrance contained in the polyurethane microcapsules at one hour. 7 out of 10 testers noticed the fragrance contained in the polyurethane microcapsules initially during use. |
| Ex. 13: Polyquaternium-10 | Poor, Initial exposure only (time = 0-1 hours) Same as above |
| Ex. 17: guar hydroxypropyltrimonium chloride N-Hance CG-17 | Good, Perfume Noticed up to 4 hours 3 out of 12 testers noticed fragrance longer than 4 hours of up to 8 hours.<br>5 out of 12 testers noticed the fragrance contained in the polyurethane microcapsules for up to 4 hours;<br>9 out of 12 testers noticed the fragrance contained in the polyurethane microcapsules initially during use. |
| Ex. 18 N-Hance CG-17+Aqupec Ser W-300C | Poor (no noticeable improvement) |

As shown in Table 2, inclusion of the cationic guar polymer, guar hydroxypropyltrimonium chloride, N-Hance CG-17 can help to provide a noticeable fragrance longevity benefit up to 4 hours or longer compared to the same product without the cationic polymer or with a different cationic polymer of inadequate polymer charge density.

As said above, Ex. 17 in view of the control showed a relatively large coacervate with a significant number of spherical clusters 2 associated with the one or more coacervates. The spherical clusters are the agglomerate of the plurality of the polyurethane microcapsules associated with the one or more coacervates. The cluster formation of the polyurethane microcapsules within the one or more coacervates in Ex. 17 can explain the relatively acceptable fragrance longevity benefit up to 4 hours or longer.

Ex. 13 and 18 have provided "poor" coacervate formation and thus have showed no fragrance longevity benefit because no spherical clusters of the polyurethane microcapsules were formed within the one or more coacervates.

Hence, the one or more coacervates may preferably comprise a plurality of clusters, wherein the plurality of clusters includes a portion of the plurality of polyurethane microcapsules. The portion of the plurality of polyurethane microcapsules may range from 5 to 50 polyurethane microcapsules per coacervated in average. Having one or more coacervates comprising a plurality of clusters which includes a portion of the plurality of polyurethane microcapsules, a fragrance longevity benefit can be obtained, namely that a fragrance originating from the plurality of polyurethane microcapsules deposited onto the skin is noticed up to 4 hours or longer after using the composition, preferably that a fragrance originating from the plurality of polyurethane microcapsules deposited onto the skin is noticed from 1 hour to 4 hours after using the composition.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

## Claims

1. A composition comprising:

    (a) an anionic surfactant system comprising from 1% to 30% of an anionic surfactant by total weight of the composition, wherein the anionic surfactant is sodium laureth(n) sulfate SLEnS, wherein n is the average moles of ethoxylation, wherein n ranges from 0 to 8, preferably from 1 to 3;
    (b) from 0.01% to 2% of a cationic guar polymer by total weight of the composition, wherein the cationic guar polymer has a charge density from 0.6 meq/g to 2.5 meq/g;
    (c) one or more coacervates formed with the anionic surfactant system, preferably with the anionic surfactant, and the cationic guar polymer;
    (d) from 0.001% to 10% of a plurality of polyurethane microcapsules by weight of the composition, wherein the polyurethane microcapsules comprise a shell material encapsulating a core material, said core material being disposed within said shell material, wherein said shell material comprises a polyurethane polymer and said core material comprises a benefit agent, wherein the polyurethane polymer of the shell material is the reaction product of the polymerization between one or more polyisocyanates and glycerine, and wherein the one or more polyisocyanates have at least two functional isocyanate groups; and

    wherein the one or more coacervates comprise one or more clusters, wherein the one or more clusters includes a portion of the plurality of polyurethane microcapsules.

2. The composition of claim 1, wherein the portion of the plurality of polyurethane microcapsules in the one or more clusters of the one or more coacervates ranges from 5 to 50, preferably from 10 to 40, more preferably from 15 to 35 polyurethane microcapsules per coacervate, as based on a visible assessment on a light micrograph, generated by a light microscope Zeiss AX10 Imager.A2 with 40X magnification from Carl Zeiss Co.; Goettengen, Germany.

3. The composition of according to any of the preceding claims, wherein the anionic surfactant system comprises from 1% to 10% of an additional anionic surfactant by total weight of the composition, wherein the additional anionic surfactant is selected from the group consisting of ammonium laureth sulfate, ammonium lauryl sulfate, triethylamine lauryl sulfate, triethylamine laureth sulfate, triethanolamine lauryl sulfate, triethanolamine laureth sulfate, monoethanolamine lauryl sulfate, monoethanolamine laureth sulfate, diethanolamine lauryl sulfate, diethanolamine laureth sulfate, lauric monoglyceride sodium sulfate, potassium laureth sulfate, sodium lauryl sarcosinate, sodium lauroyl sarcosinate, lauryl sarcosine, cocoyl sarcosine, ammonium cocoyl sulfate, sodium cocoyl isethionate, ammonium lauroyl sulfate, sodium cocoyl sulfate, sodium lauryl sulfate, potassium cocoyl sulfate, potassium lauryl sulfate, monoethanolamine cocoyl sulfate, sodium tridecyl benzene sulfonate, sodium dodecyl benzene sulfonate, and mixtures thereof.

4. The composition according to any of the preceding claims, wherein the composition comprises from 0.1% to 20% of a cosurfactant by total weight of the composition, wherein the cosurfactant is selected from the group consisting of amphoteric surfactants, zwitterionic surfactants, and mixtures thereof.

5. The composition according to claim 4, wherein the cosurfactant is a zwitterionic surfactant, preferably a betaine, more preferably cocamidopropyl betaine.

6. The composition according to any of the preceding claims, wherein the cationic guar polymer has a charge density ranging from 0.75 meq/g to 2.0 meq/g, preferably from 1.0 meq/g to 1.75 meq/g.

7. The composition according to any of the preceding claims, wherein the cationic guar polymer is a cationic guar gum derivative.

8. The composition according to claim 7, wherein the cationic guar gum derivative is guar hydroxypropyltrimonium chloride.

9. The composition according to any of the preceding claims, wherein the composition does not comprise any oil and/or lipid ingredients.

10. The composition according to any of the preceding claims, wherein the composition does not comprise any structuring anionic surfactant selecting from the group consisting of sodium trideceth(n) sulfate STnS wherein n is between 0

and 3, sodium tridecyl sulfate, sodium $C_{12}$-$_{13}$ alkyl sulfate, sodium $C_{12-15}$ alkyl sulfate, sodium $C_{11}$-$_{15}$ alkyl sulfate, sodium $C_{12}$-$_{18}$ alkyl sulfate, sodium $C_{10}$-$_{16}$ alkyl sulfate, sodium $C_{12}$-$_{13}$ pareth sulfate, sodium $C_{12}$-$_{13}$ pareth-*n* sulfate, sodium $C_{12}$-$_{14}$ pareth-*n* sulfate, and mixtures thereof.

**11.** The composition according to any of the preceding claims, wherein the composition is a micellar composition.

**12.** The composition according to any of the preceding claims, wherein the core material comprises from 6% to 99.9% of a benefit agent by total weight of the core.

**13.** The composition according to any of the preceding claims, wherein the benefit agent is selected from the group consisting of perfumes; brighteners; enzymes; perfume delivery system; conditioning agents; moisturizers; anti-microbial agents; thickeners; sensates; attractants; dyes; pigments; bleaches; and mixtures thereof.

**14.** The composition according to any of the preceding claims, wherein the polyisocyanate is isophrone diisocyanate, hexamethylene diisocyanate, trimer of hexamethylene diisocyanate, trimer of isophrone diisocyanate, or Biuret of hexamethylene diisocyanate.

**15.** The composition according to any of the preceding claims, wherein the polyurethane microcapsules have a mean diameter from 1 μm to 500 μm, preferably from 2 μm to 50 μm, more preferably from 3 μm to 30 μm.

**16.** The composition according to any of the preceding claims, wherein the composition is liquid or solid; and is the form of a hair shampoo, a hair conditioner, a liquid detergent, a fabric softener, a shower or bath cream, a body wash or foaming body wash, gel, emulsion, oil, mousse or spray, a deodorant on an antiperspirant consumer product.

**17.** Use of a composition comprising:

(a) an anionic surfactant system comprising from 1% to 30% of an anionic surfactant by total weight of the composition, wherein the anionic surfactant is sodium laureth(n) sulfate SLEnS, wherein n is the average moles of ethoxylation, wherein n ranges from 0 to 8, preferably from 1 to 3;
(b) from 0.01% to 2% of a cationic guar polymer by total weight of the composition, wherein the cationic guar polymer has a charge density from 0.6 meq/g to 2.5 meq/g;
(c) one or more coacervates formed with the anionic surfactant system, preferably with the anionic surfactant, and the cationic guar polymer; and
(d) from 0.001% to 10% of a plurality of polyurethane microcapsules by weight of the composition, wherein the polyurethane microcapsules comprise a shell material encapsulating a core material, said core material being disposed within said shell material, wherein said shell material comprises a polyurethane polymer and said core material comprises a benefit agent, wherein the polyurethane polymer of the shell material is the reaction product of the polymerization between one or more polyisocyanates and glycerine, and wherein the one or more polyisocyanates have at least two functional isocyanate groups,

wherein the one or more coacervates comprise one or more clusters, wherein the one or more clusters includes a portion of the plurality of polyurethane microcapsules;
for providing a long-lasting benefit on a surface of interest, preferably hair, skin or fabrics, more preferably hair or skin, for at least 1 hour, preferably at least 4 hours, and even at least 8 hours after initial use, preferably from 1 hour to 4 hours after initial use.

Fig. 1

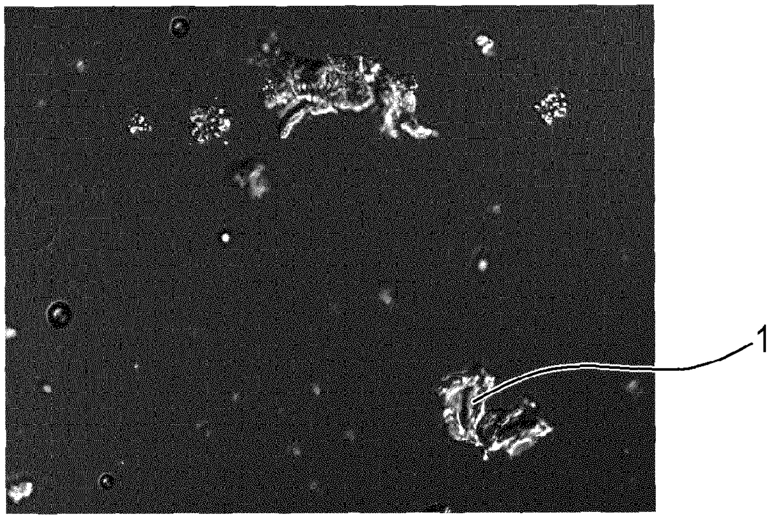


Micrograph 1: Ex. 13

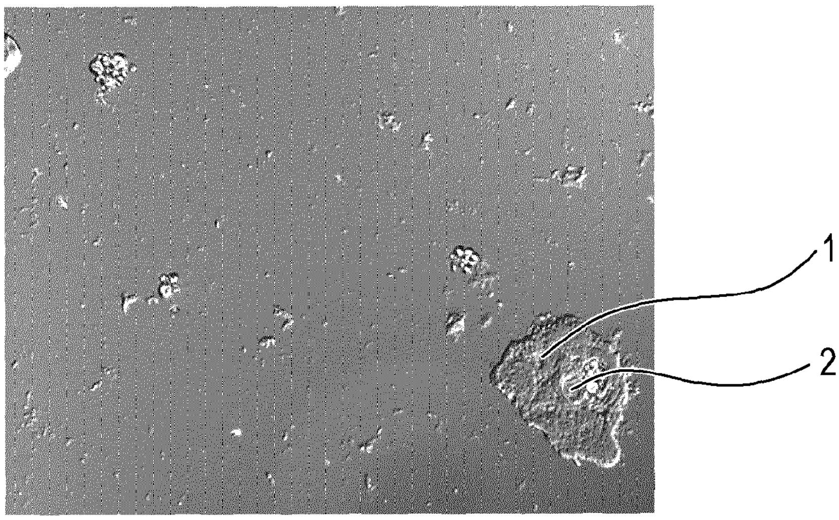


Micrograph 2: Ex. 18

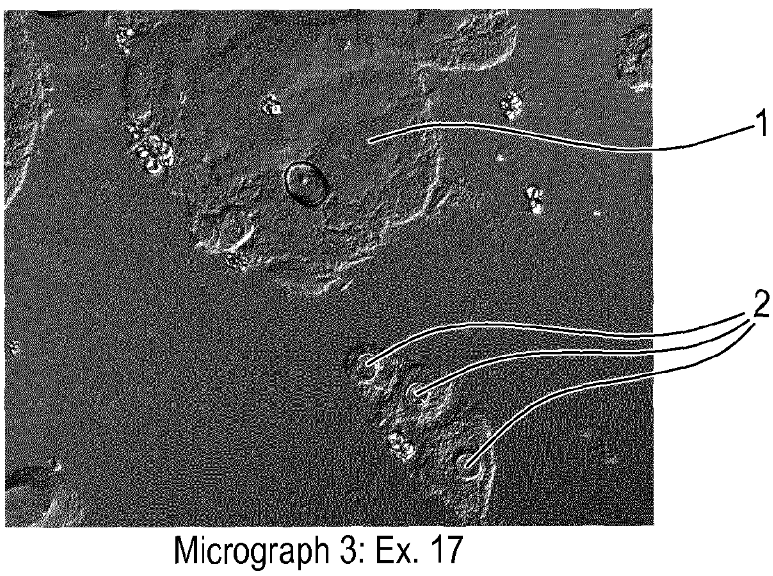


Micrograph 3: Ex. 17

**Fig. 2**

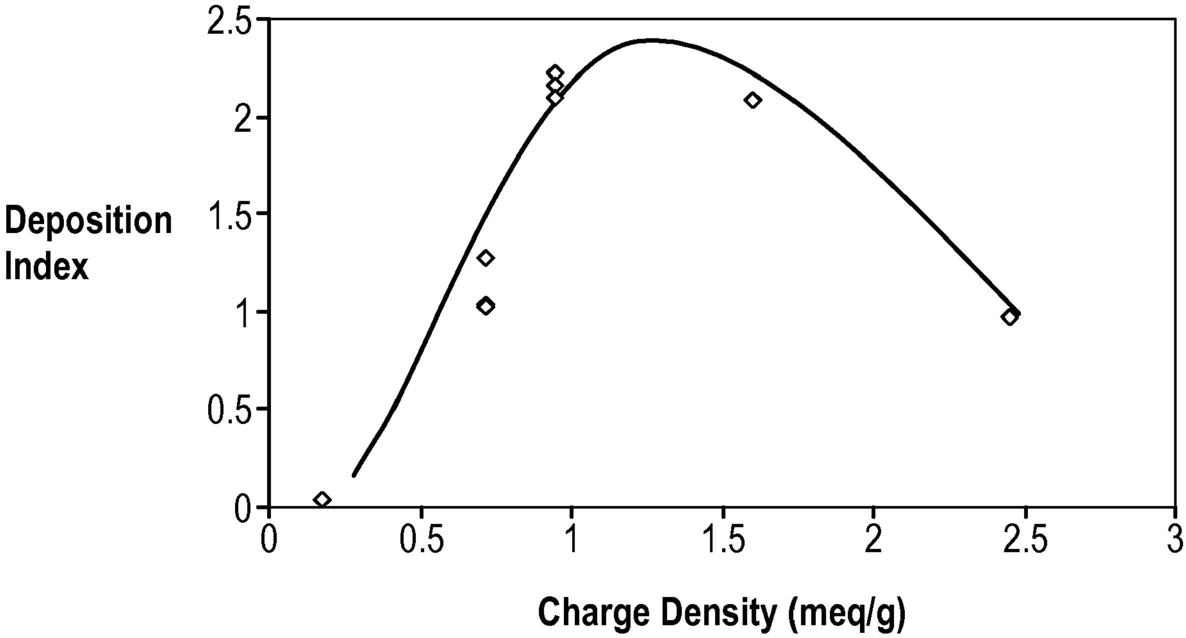
Deposition Index
2.5
2
1.5
1
0.5
0
0
0.5
1
1.5
2
2.5
3
Charge Density (meq/g)

Europäisches Patentamt
European Patent Office
Office européen des brevets

# EUROPEAN SEARCH REPORT

Application Number
EP 17 20 6592

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 2017/002293 A1 (DIHORA JITEN ODHAVJI [US] ET AL) 5 January 2017 (2017-01-05)<br>* claims 1,11 *<br>* paragraphs [0001], [0082] *<br>* examples 17B, 17G-J *<br>----- | 1-17 | INV.<br>A61K8/46<br>A61Q5/00<br>A61Q13/00<br>A61K8/73<br>A61Q19/00<br>A61K8/87<br>A61K8/11 |
| Y | US 2008/206291 A1 (OUALI LAHOUSSINE [FR] ET AL) 28 August 2008 (2008-08-28)<br>* examples 1,6 *<br>----- | 1-17 | |
| Y | US 2016/128917 A1 (WEI KARL SHIQING [US]) 12 May 2016 (2016-05-12)<br>* claims 1,9,10,15 *<br>* paragraphs [0049] - [0051], [0087] - [0090] *<br>* examples A-C, E, F, H-L *<br>* figure * *<br>----- | 1-17 | |
| Y | US 2013/089587 A1 (STAUDIGEL JAMES ANTHONY [US] ET AL) 11 April 2013 (2013-04-11)<br>* claim 1 *<br>* paragraph [0101] *<br>* Personal care compositions 1-5; tables 1,3 *<br>----- | 1-17 | TECHNICAL FIELDS SEARCHED (IPC)<br>A61K<br>A61Q |
| Y | US 2004/157754 A1 (GEARY NICHOLAS WILLIAM [US] ET AL) 12 August 2004 (2004-08-12)<br>* claims 1,4 *<br>* paragraphs [0051] - [0055] *<br>* examples 1,2,5,12,13 *<br>----- | 1-17 | |
| Y | WO 2017/071915 A1 (UNILEVER PLC [GB]; UNILEVER NV [NL]; CONOPCO INC D/B/A UNILEVER [US]) 4 May 2017 (2017-05-04)<br>* claims 1,4,5 *<br>* page 8, lines 8-9 *<br>* example * *<br>----- | 1-17 | |

The present search report has been drawn up for all claims

1

| Place of search | Date of completion of the search | Examiner |
|---|---|---|
| Munich | 7 May 2018 | Grenouillat, N |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

# ANNEX TO THE EUROPEAN SEARCH REPORT ON EUROPEAN PATENT APPLICATION NO. EP 17 20 6592

This annex lists the patent family members relating to the patent documents cited in the above-mentioned European search report. The members are as contained in the European Patent Office EDP file on
The European Patent Office is in no way liable for these particulars which are merely given for the purpose of information.

07-05-2018

| Patent document cited in search report | Publication date | Patent family member(s) | Publication date |
|---|---|---|---|
| US 2017002293 A1 | 05-01-2017 | CN 107809994 A | 16-03-2018 |
| | | EP 3316853 A1 | 09-05-2018 |
| | | US 2017002293 A1 | 05-01-2017 |
| | | WO 2017004338 A1 | 05-01-2017 |
| US 2008206291 A1 | 28-08-2008 | EP 1899047 A1 | 19-03-2008 |
| | | US 2008206291 A1 | 28-08-2008 |
| | | WO 2007004166 A1 | 11-01-2007 |
| US 2016128917 A1 | 12-05-2016 | CN 107148263 A | 08-09-2017 |
| | | EP 3217950 A1 | 20-09-2017 |
| | | US 2016128917 A1 | 12-05-2016 |
| | | WO 2016077329 A1 | 19-05-2016 |
| US 2013089587 A1 | 11-04-2013 | BR 112014007507 A2 | 04-04-2017 |
| | | CA 2850030 A1 | 11-04-2013 |
| | | CN 103841955 A | 04-06-2014 |
| | | EP 2763653 A2 | 13-08-2014 |
| | | JP 5930435 B2 | 08-06-2016 |
| | | JP 2014528454 A | 27-10-2014 |
| | | MX 339329 B | 20-05-2016 |
| | | US 2013089587 A1 | 11-04-2013 |
| | | WO 2013052771 A2 | 11-04-2013 |
| US 2004157754 A1 | 12-08-2004 | AT 507883 T | 15-05-2011 |
| | | AU 2003237505 A1 | 31-12-2003 |
| | | BR PI0311716 A2 | 27-09-2016 |
| | | CA 2487124 A1 | 24-12-2003 |
| | | CN 1662212 A | 31-08-2005 |
| | | CN 101601633 A | 16-12-2009 |
| | | EP 1513485 A2 | 16-03-2005 |
| | | ES 2365846 T3 | 11-10-2011 |
| | | HK 1076244 A1 | 18-11-2011 |
| | | JP 4049271 B2 | 20-02-2008 |
| | | JP 2005530821 A | 13-10-2005 |
| | | JP 2007277275 A | 25-10-2007 |
| | | MX PA04011709 A | 14-02-2005 |
| | | US 2004157754 A1 | 12-08-2004 |
| | | WO 03105793 A2 | 24-12-2003 |
| WO 2017071915 A1 | 04-05-2017 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5104646 A
- US 5106609 A
- US 2658072 A
- US 2438091 A
- US 2528378 A
- WO 2007004166 A1
- WO 2004078903 A
- WO 2004078901 A
- WO 2005078063 A


### Non-patent literature cited in the description


- Perfume and Flavour Chemicals. 1969
- **J. CAELLES et al.** Anionic and Cationic Compounds in Mixed Systems. *106 Cosmetics & Toiletries,* April 1991, vol. 49, 49-54
- **C. J. VAN OSS ; COACERVATION.** Complex-Coacervation and Flocculation. *9 J. Dispersion Science and Tech.,* 1988, vol. 561, 561-573
- **D. J. BURGESS.** Practical Analysis of Complex Coacervate Systems. *J. of Colloid and Interface Science,* November 1990, vol. 140 (1), 227-238
- The CTFA Cosmetic Ingredient Handbook. the Cosmetic, Toiletry, and Fragrance Association, Inc, 2004